# EUROPEAN PATENT APPLICATION

(11) **EP 4 717 297 A1**
(43) Date of publication of application: **01.04.2026**
(21) Application number: 24315443.2
(22) Date of filing: 26.09.2024
(51) Int. Cl.: A61M 25/00, A61B 17/00, A61N 1/00, A61B 17/34

(54) **SHEATH FOR SUPPORTING IMPLANTATION OF AN ELECTRODE IN THE SEPTUM OF A HUMAN HEART**

(71) Applicant: Sorin CRM SAS, 92140 Clamart (FR)
(72) Inventor: Camedda, Caroline, 35760 Saint Gregoire (FR); Cabestan, Bastienne, 8037 Zürich (CH); Russon, Austin K., South Jordan, Utah 84009 (US); Leeflang, Stephan Arie, Sandy, Utah 84092 (US)
(74) Representative: Gulde & Partner

(57) **Abstract**

A sheath (1) for supporting implantation of an electrode in the septum of a human heart, the sheath (1) comprising:

- a flexible distal end portion (2) which is at least partially curved and arranged in a two-dimensional plane (P); and

-an elongate proximal portion (3) arranged along a longitudinal axis (Z);

- wherein the flexible distal end portion (2) and the elongate proximal portion (3) are configured so that the two-dimensional plane (P) and the longitudinal axis (Z) intersect with a substantially orthogonal angle

## Description

### FIELD OF THE INVENTION

The invention relates to a sheath for implanting an electrode in the septum of a human heart, as well as a method for implantingsuch an electrode in the septum of a human heart.

### BACKGROUND OF THE INVENTION

The heart is a complex organ whose functioning is controlled by the application of precise electric fields allowing the contraction of specific muscles at specific times to regulate the filling and ejection of blood in and out of this organ.

The heart is thus a pump allowing the circulation of blood in the body. Structurally, the heart comprises four chambers (left and right atria and left and right ventricles) connected by four valves (i.e. one "tricuspid valve" between the right atrium and the right ventricle, one "mitral valve" between the left ventricle and the left atrium, one "pulmonary valve" which manages blood flow from the heart's right ventricle to the pulmonary trunk and one "aortic valve" located between the left ventricle and the aorta).

However, the functioning of the heart can sometimes be impaired, which requires the implantation of one or more medical devices delivering at the appropriate time(s) an electric current(s) to ensure the proper functioning of the organ.

Such devices (such as an implantable pacemaker and/or defibrillator also called "cardiac implantable electronic devices" or "pulse generators") typically consist of an electronic box configured for treatment by the administration of electrical current and a lead electrically connected to this box. The electronic box is typically implanted at a distance from the heart and is connected to it via the lead (typically via one of the veins supplying the heart with blood). The terms "lead", "cardiac lead", "electrode", "probe", etc. relate in the context of the present invention to the same object, i.e. an electrically conductive element, electrically isolated from its environment allowing an electrical transfer between the electronic box and the heart and/or vice versa.

Therefore, historically, right ventricular (RV) pacing has been used for pacemaker and/or defibrillator therapies of patients with cardiac deficiencies. However, in some cases, RV pacing has been shown to induce electrical and mechanical dyssynchrony.

One of the solutions adopted in recent years in the art to overcome this problem is to stimulate the septum of the heart, either by stimulating, such as a conduction system pacing, the His region or the Left Bundle Branch ("LBB"), the latter in a technic called the "LBBAP" technic (for "Left Bundle Branch Area Pacing"). These technics were thus developed to directly capture the conduction by penetrating the ventricular septum in very precise locations.

However, in such technics, the lead is typically positioned and fixed, for example via a screw, into the septum on the right ventricular side. This procedure is performed by a practitioner (such as a surgeon or electrophysiologist) and presents in particular the risk of septal perforation potentially leading to the death of the patient.

It is therefore crucial that the implantation of the lead in the heart is done precisely to allow an effective administration of the electrical treatment without jeopardizing the integrity of the heart by perforating it at the septum and threatening the life of the patient.

Thus, the practitioner who performs the implantation uses specific tools to obtain the best possible implantation.

One of these tools is a, advantageously pre-shaped, sheath (also called "catheter" in the art) in which the lead is introduced. The pre-shape of the sheath is supposed to help the practitioner find the correct placement of the lead in the heart.

For example, US 8 606 369 B2 discloses a catheter used to deliver a medical electrical lead to the right atrium of a heart in close proximity to the His bundle. In this disclosure, the catheter is adapted such that the distal tip confronts the His bundle generally perpendicularly. The catheter here includes a proximal portion and a generally hook-shaped distal portion. The distal portion may here include curves that direct the distal tip at an angle of over 180 degrees from the direction of the proximal portion.

EP 3 773 886 A1 discloses a catheter for delivering a medical electrical lead to the His bundle from within the right atrium of the heart. The catheter in this disclosure includes a straight portion and a hook portion projecting from a distal end of the straight portion. The hook portion here includes a first curved portion, a second curved portion, and a third curved portion wherein the straight portion and the first curved portion define a plane. The second curved portion extends from a distal end of the first curved portion. The second curved portion curves away from the plane. The third curved portion extends from a distal end of the second curved portion. The third curved portion curves toward the plane. The catheter forms a lumen extending from a proximal end of the straight portion to an opening at a distal end of the third curved portion.

One of the advantages of the two here-above disclosed devices is that, because the implantation is meant to be done in the right atrium, the tricuspid valve of the heart does not interfere with the elements of the implantation. However, these devices limit the scope of implantation to the His bundle, as the LBB is not easily reachable with it (lower down in the septum).

In contrast, WO 2023/099278 A1 concerns a sheath for implanting an electrode in the septum of a human heart, comprising an elongated sheath shaft which delimits a lumen. The sheath shaft has a flexible distal end portion, wherein the distal end portion is preshaped in such a way that it describes a helical curve.

The device of WO2023/099278 thus enables in theory to access the LBB region of the heart for such an implantation, enabling in particular to avoid interaction with the tricuspid valve.

### SUMMARY OF THE INVENTION

It is an objective of the present invention to ease the positioning and increase the precision of the implantation of a lead in the septum's heart of a human.

This object is achieved by sheath as claimed in claim 1 and the implementation of a method as claimed in claim 9.

According to a first aspect, it is provided a sheath for supporting implantation of an electrode in the septum of a human heart, the sheath comprising:
- a flexible distal end portion, with advantageously a radiopaque portion such as the tip (to help positioning), which is at least partially curved and arranged in a two-dimensional plane (P); and
- an elongate proximal portion arranged along a longitudinal axis (Z);
- wherein the flexible distal end portion and the elongate proximal portion are configured so that the two-dimensional plane (P) and the longitudinal axis (Z) intersect with a substantially orthogonal angle.

Surprisingly, it was discovered that such a sheath enables an easy positioning of the lead on the septum. Surprisingly, it offers a simpler design easier to implement than those of the prior art for placing a lead on the septum of a human heart. Although this valve is close from the areas of interest on the septum, the tricuspid valve bothers less the implantation thanks to the present invention. Therefore, due to its simple design, the sheath of the present invention not only facilitates septum implantations but also increases the precision of the implantations.

According to a second aspect, the invention thus relates to a method for implanting an electrode in the septum of a human heart, wherein the method comprises:
- providing a sheath according to the present invention, e.g. as the one disclosed in the first aspect here-above;
- inserting the sheath into the body of the patient and pushing the distal end portion of the sheath forward through a cardiac vein, in particular thanks for example to a dilator to straighten the catheter for insertion, so that the pre-shaped portion of the distal end portion lies on a wall of a cardiac chamber and the distal end faces in the direction of the septum within the cardiac chamber;
- pushing the electrode forward through a lumen of the sheath until it reaches the septum.

According to a first option of any one of the first or second aspects, the flexible distal end portion comprises a preshaped portion that describes a curve with a constant shape, in particular a constant radius.

According to a second option which may be combined with the first option or any one of the first or second aspects, the constant-shape curve of the distal end portion opens-up distally and/or proximally from the pre-shaped portion.

According to a third option which may be combined with the first or second option, the substantially orthogonal angle is comprised between 75° and 120°, preferably between 90 and 115°. More preferably, the substantially orthogonal angle is comprised between 92° and 110°, such as 92° ± 1°, 94° ± 1°, 100° ± 1°, 110° ± 1° or 115° ± 1°.

According to a fourth option which may be combined with any one of the first to third options or any one of the first or second aspects, the constant-shape curve is configured to wind around a parallel axis (Z1) that is substantially parallel to the longitudinal axis (Z) and thereby passes through an angular portion of a maximum of 180°, preferably less than 150°, around the parallel axis (Z1).

According to a fifth option which may be combined with any one of the first to fourth options or any one of the first or second aspects, the angle between the parallel axis (Z1) and the longitudinal axis (Z) is comprised between - 15 and + 15°. Preferably, the angle between the parallel axis (Z1) and the longitudinal axis (Z) is comprised between -10° and + 10°, more preferably between - 5° and + 5°.

According to a sixth option which may be combined with any one of the first to fifth options or any one of the first or second aspects, a shaft of the distal end portion has a soft tip at a distal end.

According to a seventh option which may be combined with any one of the first to sixth options or any one of the first or second aspects, the shaft has a stiffness that decreases towards the distal end.

According to an eighth option which may be combined with any one of the first to seventh options or any one of the first or second aspects, said sheath comprises a proximal straight portion with a stiffness comprised between a shore hardness of 65D and 85D, as measured according to ASTM D2240 or ISO 48-4, preferably between 70D and 75D, more preferably 72D.

According to a ninth option which may be combined with any one of the first to eighth options or any one of the first or second aspects, said curve presenting a portion with a decreasing stiffness with a stiffness at the distal end of said curve comprised between a shore hardness of 58D and 75D, as measured according to ASTM D2240 or ISO 48-4, preferably between 60 and 67D, more preferably 63D.

According to a tenth option which may be combined with any one of the first to ninth options or any one of the first or second aspects, said sheath comprises an end distal curve with a portion presenting a stiffness comprised between a shore hardness of 45D and 65D, as measured according to ASTM D2240 or ISO 48-4, preferably between 50D and 60D, more preferably 55D.

According to an eleventh option which may be combined with any one of the first to tenth options or any one of the first or second aspects, said sheath comprises a flexible distal end portion which is configured to retrieve a predetermined shape after a more rigid portion of a lead has completely passed through the catheter.

According to a twelfth option which may be combined with any one of the first to tenth options or any one of the first or second aspects, said sheath presents an inner diameter (i.e. defining the diameter of the lumen of the sheath which houses the lead to be implanted) comprised from 1mm to 4mm (3 French to 12 French), preferably from 1.3mm to 3mm (3.9 French to 9 French), such as about 1.83mm (about 5.5 French) or 1.65mm ± 0.1mm (4.95 French ± 0.3 French).

According to a twelfth option which may be combined with any one of the first to tenth options or any one of the first or second aspects, said sheath is slit-able, notably for an easy extraction from the patient's body.

According to a fourteenth option which may be combined with the second aspect and optionally with any one of the first to thirteenth options, a dilator is initially inserted into the lumen (L) prior to inserting the electrode.

According to a fifteenth option which may be combined with the second aspect and/or optionally with any one of the first to fourteenth options, the electrode is a pacemaker electrode and/or a defibrillator electrode.

According to a sixteenth option which may be combined with the second aspect and/or optionally with any one of the first to fifteenth options, the pacemaker electrode and/or a defibrillator electrode is implanted at a location suitable for His bundle and/or LBB stimulation. In particular, the His bundle and/or LBB stimulation is a conduction system pacing, i.e. a direct activation of the conduction system of the heart by the pacing stimulus, comprising His bundle and/or LBBAP deep septum.

It shall be understood that the sheath of claim 1 and the method of claim 9, may comprise similar and/or identical preferred embodiments, in particular, as defined in the dependent claims.

It shall be understood that a preferred embodiment of the invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 represents an overview of a sheath according to the present invention;
Fig. 2 is a first view of a first embodiment of a sheath according to the present invention;
Fig. 3 is a second view the first embodiment of a sheath according to figure 2;
Fig. 4 is a first view of a second embodiment of a sheath according to the present invention;
Fig. 5 is a second view the second embodiment of a sheath according to figure 4;
Fig. 6 is a first view of a third embodiment of a sheath according to the present invention;
Fig. 7 is a second view the third embodiment of a sheath according to figure 6;
Fig. 8 is a first view of a fourth embodiment of a sheath according to the present invention;
Fig. 9 is a second view the fourth embodiment of a sheath according to figure 8;
Fig. 10 is a first view of a fifth embodiment of a sheath according to the present invention;
Fig. 11 is a second view the fifth embodiment of a sheath according to figure 10;

### DETAILED DESCRIPTION OF EMBODIMENTS

Various embodiments of the present invention are now described based on a sheath for implanting a lead on the septum of a heart, preferably at a location suitable for His bundle and/or LBB stimulation.

It is noted that - throughout the present disclosure - only those structural elements and functions are shown and/or described, which are useful to understand the embodiments. Other structural elements and functions are omitted for brevity reasons. Furthermore, the structure and/or function of blocks, elements or components with identical reference numbers that have been described before are not described again, unless an additional specific functionality is involved.

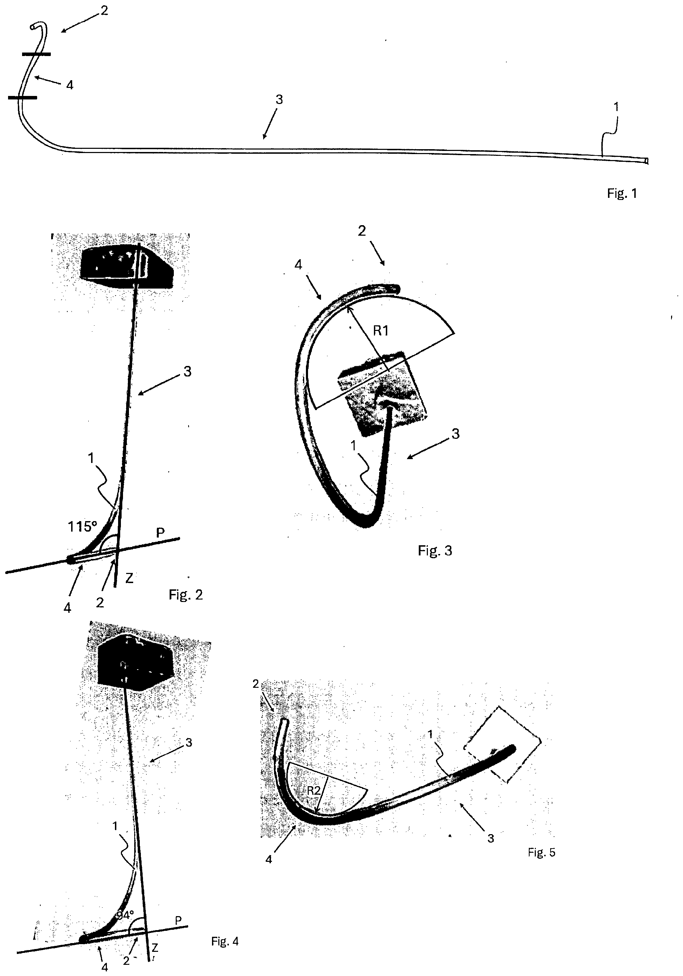

Fig. 1 represents a sheath 1 according to the present invention, with a flexible distal end portion 2, an elongate proximal portion 3 and a preshaped portion 4 that describes a curve with a constant shape, advantageously constant radius.

For example, in Fig. 1, the sheath 1 comprises an elongate proximal portion 3 with a stiffness (shore hardness) comprised between 65D and 85D, as measured according to ASTM D2240 (ISO 48-4), such as 72D, which then decreases, in particular up to about halfway through this primary curve, to a stiffness comprised between 58D and 75D, such as 63D, at the preshaped portion 4, and stay constant until the end of this first curve in order to then decrease to a smooth transition to a stiffness comprised between 45D and 65D, such as 55D, used for the flexible distal end portion 2 as a secondary curve as illustrated. The sheaths as disclosed present typical lengths comprised between 35 and 50 cm, preferably between 40 and 47 cm, such as between 41 and 45 cm.

The Shore D Hardness Scale measures the hardness of hard rubbers, semirigid plastics and hard plastics. Shore hardness (durometer) is a measure of a material's resistance to indentation.

Shore hardness, using the Shore A or Shore D scale, is the preferred method for rubbers and thermoplastic elastomers - and is also commonly used for "softer" plastics such as polyolefins, fluoropolymers, and vinyls.

Different types of instruments exist, and the Shore A and IRHD Micro/Dead Load scales are the most commonly used for rubber. International standards describe both methods, namely ISO 48-4:2018, *"Rubber, vulcanized or thermoplastic* - *Determination of hardness"* and ASTM D2240-15 (2021), *"Standard Test Method for Rubber Property - Durometer Hardness".*

Fig. 2 and 3 represent different views of a first embodiment of a sheath 1 according to the present invention, wherein an elongate proximal portion 3 is arranged along a longitudinal axis Z, a flexible distal end portion 2 is partially curved and arranged in a two-dimensional plane P, and a preshaped portion 4 describes a curve with a constant radius R1. The two-dimensional plane P and the longitudinal axis Z intersect with a substantially orthogonal angle of 115°. This first embodiment could be used for smaller hearts, with a quite high angled primary curve (the first curve directly linked to the elongate proximal portion 3 and which diverts the sheath away from the longitudinal axis Z), also allowing to reach a more basal position in normal hearts. This first embodiment would not be the "standard" shape for common hearts. For example, this sheath could present an inner diameter (i.e. defining the diameter of the lumen of the sheath which houses the lead to be implanted) comprised from 1mm to 4mm (3 French to 12 French), preferably from 1.3mm to 3mm (3.9 French to 9 French), such as about 1.83mm (about 5.5 French) or 1.65mm ± 0.1mm (4.95 French ± 0.3 French). For example, this sheath could present an outer diameter (i.e. defining the outer diameter of the cross section of the sheath which defines the outer surface of the sheath) comprised from 1.8mm to 6mm (5.4 French to 18 French), preferably from 2mm to 4mm (6 French to 12 French), such as about 2.54mm (about 7.5 French). For example, the wall thickness of the sheath could be comprised from 0.2mm to 3mm, preferably from 0.3mm to 1mm, such as about 0.37mm ± 0.2mm.

Fig. 4 and 5 represent different views of a second embodiment of a sheath 1 according to the present invention, wherein an elongate proximal portion 3 is arranged along a longitudinal axis Z, a flexible distal end portion 2 is partially curved and arranged in a two-dimensional plane P, and a preshaped portion 4 describes a curve with a constant radius R2. The two-dimensional plane P and the longitudinal axis Z intersect with a substantially orthogonal angle of 94°. This second embodiment was typically designed for common size hearts, able to easily reach the septum, notably the LBB corresponding region(s) of normal hearts. This second embodiment could be used as the "standard" shape for common hearts. For example, this sheath could present an inner diameter (i.e. defining the diameter of the lumen of the sheath which houses the lead to be implanted) comprised from 1mm to 4mm (3 French to 12 French), preferably from 1.3mm to 3mm (3.9 French to 9 French), such as about 1.83mm (about 5.5 French) or 1.65mm ± 0.1mm (4.95 French ± 0.3 French). For example, this sheath could present an outer diameter (i.e. defining the outer diameter of the cross section of the sheath which defines the outer surface of the sheath) comprised from 1.8mm to 6mm (5.4 French to 18 French), preferably from 2mm to 4mm (6 French to 12 French), such as about 2.54mm (about 7.5 French). For example, the wall thickness of the sheath could be comprised from 0.2mm to 3mm, preferably from 0.3mm to 1mm, such as about 0.37mm ± 0.2mm.

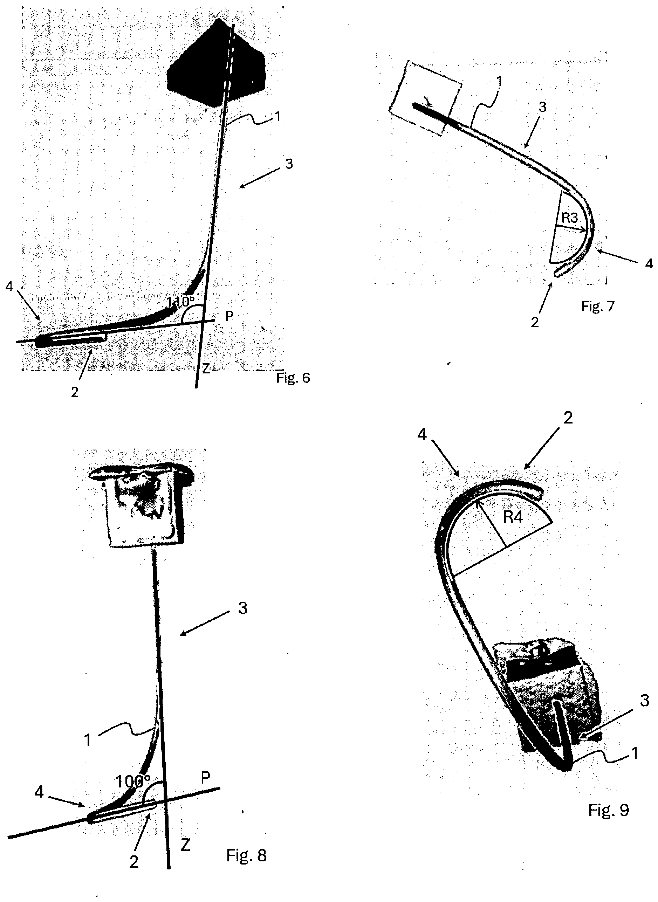

Fig. 6 and 7 represent different views of a third embodiment of a sheath 1 according to the present invention, wherein an elongate proximal portion 3 is arranged along a longitudinal axis Z, a flexible distal end portion 2 is partially curved and arranged in a two-dimensional plane P, and a preshaped portion 4 describes a curve with a constant radius R3. The two-dimensional plane P and the longitudinal axis Z intersect with a substantially orthogonal angle of 110°. This third embodiment could be used for dilated hearts. For example, this sheath could present an inner diameter (i.e. defining the diameter of the lumen of the sheath which houses the lead to be implanted) comprised from 1mm to 4mm (3 French to 12 French), preferably from 1.3mm to 3mm (3.9 French to 9 French), such as about 1.83mm (about 5.5 French) or 1.65mm ± 0.1mm (4.95 French ± 0.3 French). For example, this sheath could present an outer diameter (i.e. defining the outer diameter of the cross section of the sheath which defines the outer surface of the sheath) comprised from 1.8mm to 6mm (5.4 French to 18 French), preferably from 2mm to 4mm (6 French to 12 French), such as about 2.54mm (about 7.5 French). For example, the wall thickness of the sheath could be comprised from 0.2mm to 3mm, preferably from 0.3mm to 1mm, such as about 0.37mm ± 0.2mm.

Fig. 8 and 9 represent different views of a fourth embodiment of a sheath 1 according to the present invention, wherein an elongate proximal portion 3 is arranged along a longitudinal axis Z, a flexible distal end portion 2 is partially curved and arranged in a two-dimensional plane P, and a preshaped portion 4 describes a curve with a constant radius R4. The two-dimensional plane P and the longitudinal axis Z intersect with a substantially orthogonal angle of 110°. This fourth embodiment could be used for common size hearts, able to easily reach the septum, notably the LBB corresponding region(s) of normal hearts wherein a mandril is used. The mandril is different but for the final shape of the catheter is the same when the lead is inserted. This fourth embodiment could be used as the "standard" shape for common hearts when a mandril is used. For example, this sheath could present an inner diameter (i.e. defining the diameter of the lumen of the sheath which houses the lead to be implanted) slightly wider than the one Fig. 4 and 5 comprised from 1.5mm to 5mm (4.5 French to 15 French), preferably from 2mm to 4mm (6 French to 12 French), such as about 2.4892mm (about 7.5 French). For example, this sheath could present an outer diameter (i.e. defining the outer diameter of the cross section of the sheath which defines the outer surface of the sheath) comprised from 2.3mm to 8mm (6.9 French to 24 French), preferably from 2.6mm to 3.7mm (7.8 French to 11.1 French), such as about 3.10mm (about 9.3 French). For example, the wall thickness of the sheath could be comprised from 0.1mm to 2mm, preferably from 0.2mm to 1mm, such as about 0.30mm ± 0.1mm.

Fig. 10 and 11 represent different views of a fifth embodiment of a sheath 1 according to the present invention, wherein an elongate proximal portion 3 is arranged along a longitudinal axis Z, a flexible distal end portion 2 is partially curved and arranged in a two-dimensional plane P, and a preshaped portion 4 describes a curve with a constant radius R5. The two-dimensional plane P and the longitudinal axis Z intersect with a substantially orthogonal angle of 100°. This fifth embodiment could be used for smaller hearts, with a quite high angled primary curve (the first curve directly linked to the elongate proximal portion 3 and which diverts the sheath away from the longitudinal axis Z), able to reach a more basal position than for normal hearts, wherein a mandril is used. The mandril is different but for the final shape of the catheter is the same when the lead is inserted. For example, this sheath could present an inner diameter (i.e. defining the diameter of the lumen of the sheath which houses the lead to be implanted) slightly wider than the one figures 4 and 5 comprised from 1.5mm to 5mm (4.5 French to 15 French), preferably from 2mm to 4mm (6 French to 12 French), such as about 2.4892mm (about 7.5 French). For example, this sheath could present an outer diameter (i.e. defining the outer diameter of the cross section of the sheath which defines the outer surface of the sheath) comprised from 2.3mm to 8mm (6.9 French to 24 French), preferably from 2.6mm to 3.7mm (7.8 French to 11.1 French), such as about 3.10mm (about 9.3 French). For example, the wall thickness of the sheath could be comprised from 0.1mm to 2mm, preferably from 0.2mm to 1mm, such as about 0.30mm ± 0.1mm.

### EXAMPLES

Comparative examples were made with other products of competitors. Such conventional catheters, present a shape roughly in one sole plane with a curve distal end. The overall design of such conventional catheters resembles that of a hook as illustrated in the US 8 606 369 B2. Such conventional catheters are built on the catheter design for His bundle stimulation implantation.
The design of the present embodiments proved in the realized tests that the catheters were easier to position, more stable once in position and presented higher stability after torque at position for equivalent designs as those of conventional catheters.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to -advantage. The foregoing description details certain embodiments of the invention. It will be appreciated, however, that no matter how detailed the foregoing appears in the text, the invention may be practiced in many ways, and is therefore not limited to the embodiments disclosed. It should be noted that the use of particular terminology when describing certain features or aspects of the invention should not be taken to imply that the terminology is being re-defined herein to be restricted to include any specific characteristics of the features or aspects of the invention with which that terminology is associated.

## Claims

1. A sheath (1) for supporting implantation of an electrode in the septum of a human heart, the sheath (1) comprising:
- a flexible distal end portion (2) which is at least partially curved and arranged in a two-dimensional plane (P); and
- an elongate proximal portion (3) arranged along a longitudinal axis (Z);
- wherein the flexible distal end portion (2) and the elongate proximal portion (3) are configured so that the two-dimensional plane (P) and the longitudinal axis (Z) intersect with a substantially orthogonal angle.

2. The sheath (1) according to claim 1, wherein the flexible distal end portion (2) comprises a preshaped portion (4) that describes a curve with a constant shape, in particular a constant radius.

3. The sheath (1) according to claim 2, wherein the constant-shape curve of the distal end portion opens-up distally and/or proximally from the pre-shaped portion.

4. The sheath (1) according to any one of the preceding claims, wherein the substantially orthogonal angle is comprised between 75° and 120°, preferably between 90 and 115°.

5. The sheath (1) according to any one of the preceding claims, wherein the constant-shape curve is configured to wind around a parallel axis (Z1) that is substantially parallel to the longitudinal axis (Z) and thereby passes through an angular portion of a maximum of 180°, preferably less than 150°, around the parallel axis (Z1).

6. The sheath (1) according to claim 5, wherein the angle between the parallel axis (Z1) and the longitudinal axis (Z) is comprised between -15 and +15°.

7. The sheath (1) according to any one of the preceding claims, wherein a shaft of the distal end portion has a soft tip at a distal end.

8. The sheath (1) according to claim 7 wherein the shaft has a stiffness that decreases towards the distal end.

9. The sheath (1) according to any one of the preceding claims, wherein said sheath (1) comprises a proximal straight portion with a stiffness comprised between 65D and 85D, as measured according to ASTM D2240 or ISO 48-4, preferably 72D.

10. The sheath (1) according to any one of the preceding claims, wherein said sheath (1) comprises a curve directly linked to a proximal straight portion, said curve presenting a portion with a decreasing stiffness with a stiffness at the distal end of said curve comprised between 58D and 75D, as measured according to ASTM D2240 or ISO 48-4, preferably 63D.

11. The sheath (1) according to any one of the preceding claims, wherein said sheath (1) comprises an end distal curve with a portion presenting a stiffness comprised between 45D and 65D, as measured according to ASTM D2240 or ISO 48-4, preferably 55D.

12. A method for implanting an electrode in the septum of a human heart, wherein the method comprises:
- providing a sheath (1) according to one of the preceding claims;
- inserting the sheath (1) into the body of the patient and pushing the distal end portion of the sheath (1) forward through a cardiac vein so that the pre-shaped portion of the distal end portion lies on a wall of a cardiac chamber and the distal end faces in the direction of the septum within the cardiac chamber;
- pushing the electrode forward through a lumen of the sheath (1) until it reaches the septum.

13. The method according to claim 12, wherein a dilator is initially inserted into the lumen (L) prior to inserting the electrode.

14. The method according to claim 12 or 13, wherein the electrode is a pacemaker electrode and/or a defibrillator electrode.

15. The method according to claim 14, wherein the pacemaker electrode and/or a defibrillator electrode is implanted at a location suitable for His bundle and/or LBB stimulation.
